# EUROPEAN PATENT APPLICATION

(11) **EP 4 035 692 A1**
(43) Date of publication of application: **03.08.2022**
(21) Application number: 22153564.4
(22) Date of filing: 27.01.2022
(51) Int. Cl.: A61L 2/10, H01L 25/00, H01L 33/58, F21V 5/00, G02B 6/00, G02B 5/00

(54) **SANITIZING LAMP**

(30) Priority: 27.01.2021 IT 202100001538
(71) Applicant: Ugolini & C. S.r.l., 50028 Barberino Tavarnelle (FI) (IT)
(72) Inventor: MESSINA, Gabriele, 50028 Barberino Tavarnelle, FIRENZE (IT); UGOLINI, Bianca Maria, 50028 Barberino Tavarnelle, FIRENZE (IT); CEVENINI, Gabriele, 50028 Barberino Tavarnelle, FIRENZE (IT)
(74) Representative: Long, Giorgio

(57) **Abstract**

The present invention relates to a sanitizing lamp, in particular a room lamp.

In particular, the invention relates to a sanitizing lamp (1, 101) comprising:
- at least one lighting module (2) equipped with a plurality of white light LED sources (6) and a plurality of blue light LED sources (7);
- a diffuser screen (3),
- a frame (4) and
- a suspension element (5),
**characterized in that** the diffuser screen (3) consists of a sheet of transparent plastic material having a refractive index between 1.4 and 1.6, a light transmittance greater than 90%, and a haze <0.600%, and comprises a smooth outer face (3a) and an inner face (3b) with a hexagonal honeycomb structure.

## Description

The present invention relates to a sanitizing lamp, in particular a room lamp.

In an increasingly globalized and populated world, the spread of bacterial and viral infections may represent an important risk for the population. The recent SARS-COV 2 pandemic has highlighted the critical issues related to the easy spread of an infection and the difficulty of effectively combating it with the currently available means.

The use of disinfectants for surfaces and body parts, as well as personal hygiene or social distancing, while being of certain usefulness, are nevertheless linked to individual behaviors which are not always easy to implement or verify.

Even the use of masks, although not decisive, provides a valuable aid in reducing bacterial or viral transmission, but once again it depends on the willingness and ability of people to wear them correctly and continuously.

The restrictions which many categories of workers have had to implement further entail serious economic consequences, which could be overcome if tools were available to achieve a safe workplace and/or public environment. The same stands in the field of education, with a view to the continuity of frontal teaching which is undoubtedly a primary objective.

Disinfection by means of ultraviolet (UV) rays in the C range (wavelengths between 100 and 280 nm) has been a consolidated practice for years, due to the biocidal properties of UV-C radiation and the advantages that physical techniques have with respect to chemical techniques. There are many current applications on air, water and surfaces. Both UV-C lamps with high luminous power (over 100 W) as well as increasingly powerful LED sources are now available on the market.

The extreme flexibility of the latter has favored the widespread use of LED sources at least in particular fields, where the distances from the UV source are smaller, and consequently they are used on spaces, surfaces and objects to be disinfected of a more limited size. In fact, it is known that radiant energy decreases with the square of the distance.

However, UV-C radiation is harmful to the skin or eyes of a person subjected to such radiations, therefore the use thereof is limited to specific applications.

Therefore, the problem underlying the present invention is the provision of a disinfectant lamp which provides a good abatement of the bacterial and/or viral load in an environment and on the surfaces present while being able to be operated even in the presence of one or more persons.

Such a problem is overcome by a sanitizing lamp as defined in the appended claims, the definitions of which form an integral part of the present description.

The present invention relates to a sanitizing lamp comprising:
- at least one lighting module equipped with a plurality of white light LED sources and a plurality of blue light LED sources;
- a diffuser screen,
- a frame and
- a suspension element,
**characterized in that** the diffuser screen consists of a sheet of transparent plastic material having a refractive index between 1.4 and 1.6, a light transmittance over 90%, and a haze <0.600%, and comprises a smooth outer face and an inner face with a hexagonal honeycomb structure.

Further features and advantages of the present invention will become more apparent from the description of certain embodiments thereof, given hereafter only by way of a non-limiting, indicative example, with reference to the following figures:
Figure 1 shows a perspective view of a ceiling lamp according to the invention;
Figure 2 shows a perspective view of a suspension lamp according to the invention;
Figure 3 shows a partial photographic plan view of a detail of the lamp in Figure 1 or 2;
Figure 4 shows a partial diagrammatic plan view of the detail in Figure 3;
Figure 4A shows a diagrammatic cross-section view of a detail in Figure 4;
Figure 5 shows a plan view of the lighting scheme of the lamp in Figure 1 or 2;
Figure 6 shows a cross-section view of the lamp in Figure 1;
Figure 7 shows a detail of the lighting scheme in Figure 5.

The sanitizing lamp according to the invention, indicated as a whole by numeral 1, comprises:
- at least one lighting module 2 equipped with a plurality of white light LED sources and a plurality of blue light LED sources;
- a diffuser screen 3,
- a frame 4 and
- a suspension element.

The term "blue light" generally means an electromagnetic radiation with a wavelength between 380 and 500 nm and which appears blue to the eye. More in particular, for the purposes of the present invention, the term "blue light" most preferably consists of an electromagnetic radiation with a wavelength between 400 and 410 nm and with a peak of about 405 nm.

Lamp 1 combines a classic lighting effect - given by the white light LED sources - with an antibacterial and antiviral effect, given by the blue light LED sources. It has been observed that such an antibacterial and antiviral effect occurs not only on the lighted surfaces, but also on the bacterial/viral load present in the air.

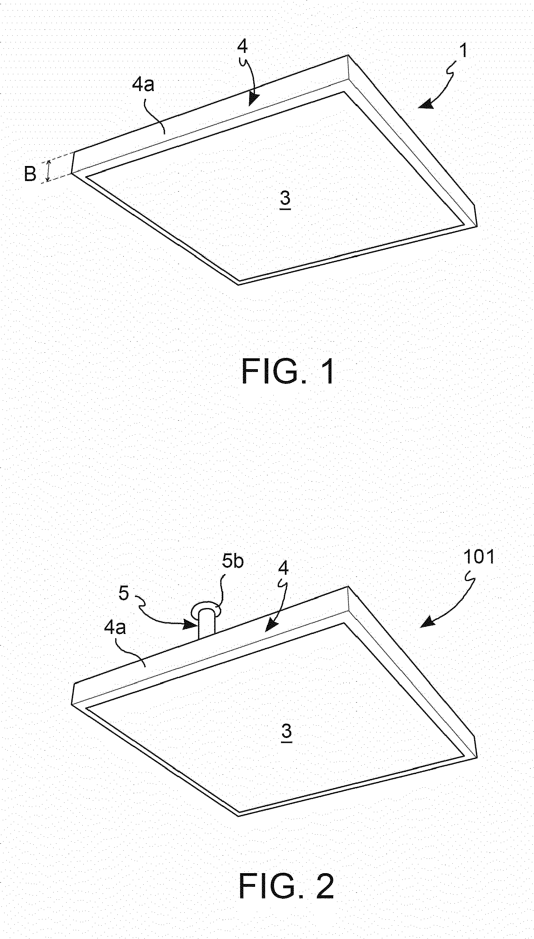

In the embodiment shown in Figure 1, the sanitizing lamp according to the invention is a ceiling light, while in the embodiment shown in Figure 2 it is a suspension lamp. However, in other embodiments, the sanitizing lamp may be a floor lamp, a table lamp, or a wall lamp (a so-called "applique") or the like.

Therefore, the term "suspension element" means both an element for fastening the lamp to a ceiling or to a wall, as well as a suspension arm for a table lamp or a floor lamp, and a stem for a suspension lamp as such.

The shape of the lamp 1, 101 shown in the Figures is merely indicative, since the lamp may have any shape, for example, rectangular, circular, elliptical, triangular and polygonal in general.

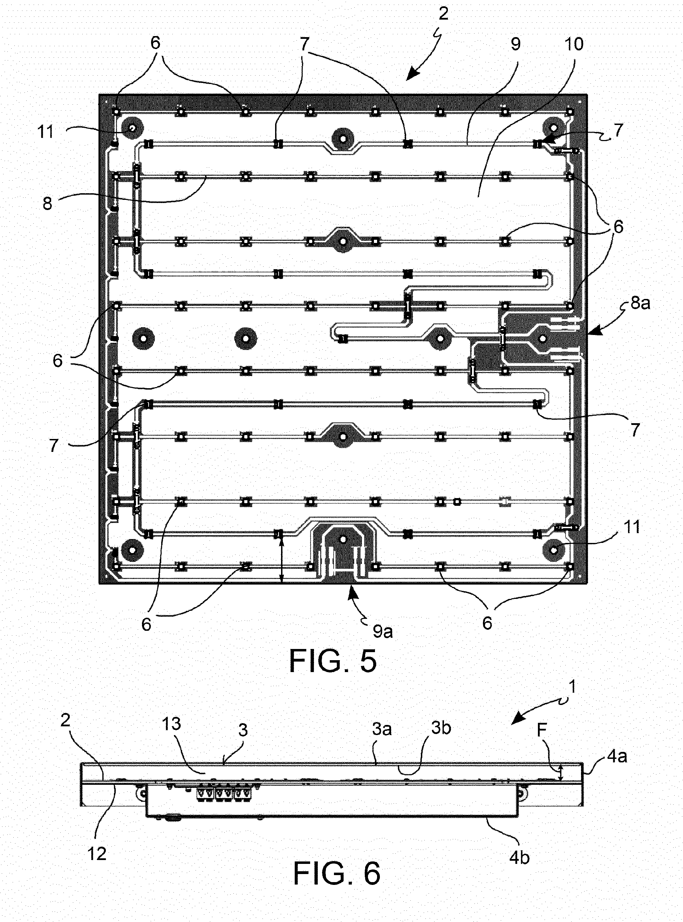

The lamp may also be of the most varied size. By way of example, the lighting module 2 shown in Figure 5 is configured for a lamp of a size of 30 cm × 30 cm. Two or more lighting modules 2 may be placed side-by-side in the case of lamps of a larger size. For example, a lamp with a size of 60 cm × 60 cm will comprise four lighting modules 2, while a lamp with a size of 30 cm × 60 cm will comprise two lighting modules 2. The size of the lamp will generally be dictated by the spatial needs of the environment in which it will be installed. It will also be possible to prepare a set of sanitizing lamps 1, 101 arranged in a regular manner, so as to irradiate the entire space of the environment.

The frame 4 may be made of any material suitable for the construction of lamps. For example, the frame 4 may be made of wood, plastic material, metal such as, for example, aluminum, steel, copper or bronze, or a combination of such materials.

The frame 4 comprises an edge 4a and a support plate 4b. Preferably, the support plate 4b will be made of metal, while the edge 4a may be made of the materials listed above, mainly selected based on aesthetic reasons.

The edge 4a comprises fastening means (not visible) for the diffuser screen 3, which may be slightly recessed, flush or protruding with respect to the edge 4a.

In the embodiment in Figure 2, the suspension element 5 comprises a stem 5a and an element 5b for the fastening to the ceiling of an environment. The support plate 4b is fastened to the stem 5a of the suspension element 5.

It is preferable that the mutually facing surfaces of the lighting module 2 and the diffuser screen 3 are spaced apart so as to create a spacing 13 therebetween with a height F of between 3 and 4.5 cm, more preferably of about 4 cm. The spacing 13 is useful for dissipating the heat generated by the LEDs. Therefore, the height B of the edge 4a of the frame 4 will be designed so as to allow establishing such a distance upon fastening the lighting module 2 on the support plate 4b and the diffuser screen 3 on the edge 4a.

It has also been found that the optimal treatment distance between the lighting module 2 and the surfaces to be treated and/or the space to be treated between such surfaces and the lamp 1, 101 is 2 meters or less. When such a distance is 2 meters or less, the sanitizing effect is maximized. Therefore, taking into account that the surfaces to be sanitized are preferably arranged between 40 cm (seats) and 90 cm (tables, kitchen shelves, etc.) and that the height of the ceiling from the floor is generally variable from a minimum of 2.40 meters to more, the sanitizing lamp 1, 101 will preferably be chosen according to such a height. For example, in the case of a ceiling with a height between 2.40 and 2.90 meters, a ceiling light (sanitizing lamp 1) may also be used, while for higher heights a suspension lamp 101 will be preferably used, in which the stem 5a of the suspension element 5 will have a length such as to be able to establish the treatment distance described above. In particular, if L is the distance between the ceiling and the floor, the length Lg of the stem 5a in meters will preferably be given by the equation Lg = L - x, x being between 2.40 and 2.90 meters.

The lighting module 2, shown in Figure 5, comprises a plurality of white light emitting LEDs 6 (briefly defined as "white LEDs") and a plurality of blue light emitting LEDs 7 (briefly defined as "blue LEDs") .

The lighting module 2 further comprises a power supply circuit 8 for the white LEDs 6 and a power supply circuit 9 for the blue LEDs 7.

The power supply circuit 8 for the white LEDs 6 is connected to a power supply connector 8a, while the power supply circuit 9 for the blue LEDs 7 is connected to a power supply connector 9a. Thereby, it will be possible to selectively switch on the white LEDs 6 and/or the blue LEDs 7, either by means of a switch, or by means of suitable light sensors.

The aforesaid circuits 8, 9 and the LEDs 6, 7 are supported on a panel 10 made of insulating material, which comprises a plurality of holes 11 for the fastening to a heat-dissipating plate 12.

The heat-dissipating plate 12 preferably consists of an aluminum plate with a thickness of about 3 mm. Such a thickness is a good compromise between the sufficient amount of heat dissipated and the weight (as well as the associated cost) of the plate. The heat-dissipating plate 12 acts in combination with the spacing 13 between the lighting module 2 and the diffuser screen 3 to dispose of the heat generated by the LEDs and thus improve the efficiency thereof.

The blue LEDs 7 emit light at a wavelength between 400 and 410 nm, with a peak at 405 nm, as specified above.

It has been seen that at such a wavelength the antibacterial and antiviral effect of the UV LEDs is maintained (albeit with longer treatment times), but without creating skin damage to a person irradiated by such a light. Therefore, the lamp 1 according to the invention may be kept on even in the presence of persons operating in the treated environment, thus ensuring a continuous sanitization of such an environment.

Preferably, the blue LEDs 7 are characterized by a minimum radiometric flux between 900 and 1000 mW, which may be driven in current so as to obtain an effective radiometric flux between 1000 and 1500 mW. For example, it will be possible to apply a current intensity between 700 and 900 mA with a voltage between 5 and 6 Volts.

Preferably, the blue LEDs 7 are configured to generate a lighting cone with a vertex angle of about 130°.

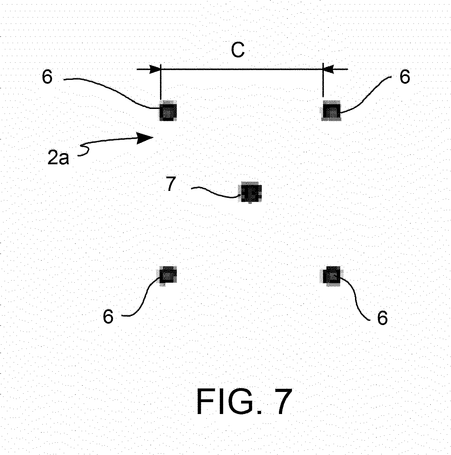

Figure 7 shows a detail of the arrangement of the white LEDs 6 and the blue LEDs 7. The lighting module 2 consists of a plurality of lighting units 2a consisting of four white LEDs 6 arranged at the vertices of a square and one blue LED 7 arranged in the center of the square. Preferably, the side C of the square has a length of about 35 mm.

Such an arrangement allows masking the blue light, which is visually unpleasant, by virtue of the white light emitted by the white LEDs 6 surrounding the blue LEDs 7. The white light preferably used has a spectrum in which the blue component is reduced to a minimum: a good choice is neutral white light (neither warm nor cold) at a temperature close to 3000 degrees Kelvin.

It is also important that the distance D of the outermost blue LEDs 7 from the edge 4a is 5 cm or more, preferably about 6 cm. Thereby, the edge 4a may be prevented from partially shielding the lighting cone of the blue LEDs 7.

In a particularly preferred embodiment of the invention, the lighting module 2 comprises 64 white LEDs 6 and 17 blue LEDs 7.

The diffuser screen 3 consists of a sheet of transparent plastic material having a refractive index between 1.4 and 1.6, a light transmittance over 90% and a haze <0.600%. Preferably, the plastic material is polymethylmethacrylate (PMMA) preferably having a density of 1.19 g/cc and the following optical properties:

| | |
|---|---|
| - refractive index | 1.490 |
| - light transmittance | 93.0% |
| - haze | <0.500%. |

Such a material is for example commercially available under the brand SUMIPEX^{®} EX of Sumitomo Chemical Co., Ltd..

Preferably, the diffuser screen 3 has a thickness of between 2 and 4 mm, more preferably of about 3 mm.

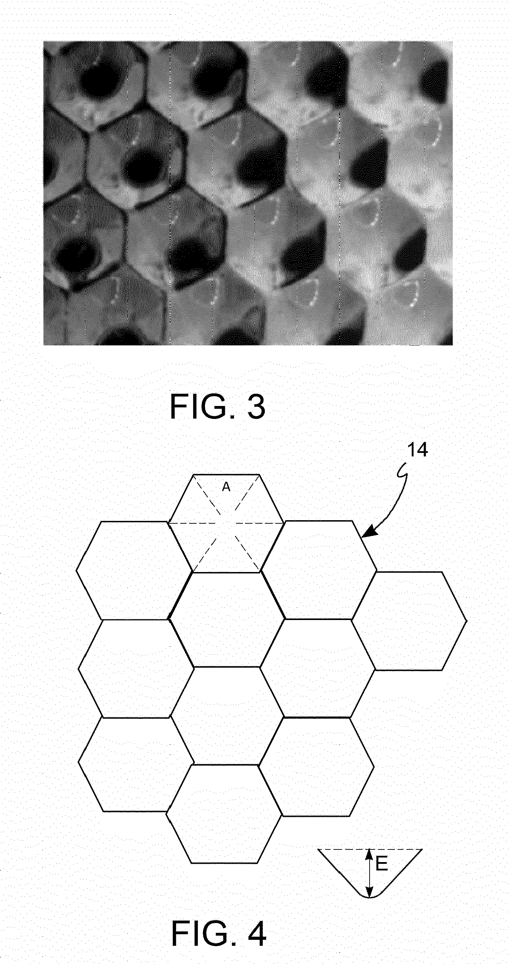

The diffuser screen 3 comprises a smooth outer face 3a and an inner face 3b with a hexagonal honeycomb structure. As shown in the photograph in Figure 3 and in the drawing in Figure 4-4A, such a honeycomb structure consists of hexagons 14 placed side-by-side, where each hexagon 14 has a side A of a length between 0.8 and 0.9 mm and a profile flared towards the inside with a depth E between 0.4 and 0.5 mm.

It has been seen that the honeycomb structure described above provides, on the one hand, an adequate and uniform diffusion of the light emitted by the white LEDs 6 and by the blue LEDs 7, an indispensable feature for the use in the presence of people, and, on the other hand, a transmission of the blue radiation (400-410 nm) which is slightly lower than that of a smooth transparent sheet made of the same material.

Table I below shows a comparison between different diffuser screens made with the same material (PMMA having the optical properties listed above), but with a different surface finish.

The test was conducted with LED emitters with 400<λ<410 nm (operating conditions: E=5.4 V, I=80 mA), placing the diffuser screen 3 at a distance of 4 cm from the blue LED sources 7 and the detection probe 6 cm from such LED sources.

Reference values recorded without panel in the range 380<λ<430 nm:
- Distance from the probe = 6 cm
- Radiant flux = 5.24 mW (detected by the probe)
- Photons = 1.85E-5 µMol (detected by the probe).

The probe used is of the cosine corrector type connected to the spectrophotometer by means of a special fiber optic cable.

**Table I - Radiant flux and photonic transmission of LED light at a wavelength of 400-410 nm by means of a diffuser screen**

| **Screen finish** | **Screen thickness (mm)** | **Radiant flux (mW)** | **Photons (**µMol**)** |
|---|---|---|---|
| Glazed¹ | 2 | 2.90 | 1.05E-5 |
| transparent | 4 | 4.86 | 1.74E-5 |
| prismatic honeycomb² | 3 | 2.907 | 1.03E-5 |
| hexagonal honeycomb³ | 3 | 4.54 | 1.67E-5 |

| | | | |
|---|---|---|---|
| ¹ light transmittance 71% ² prisms with side of 2.1 mm ³ hexagons with side of 0.83 mm. | | | |

As it may be seen from the above data, the hexagonal honeycomb structure according to the invention allows obtaining a transmission of blue radiation (400-410 nm) which is slightly lower than that of a transparent screen, which however is not suitable for diffusing light in an environment in a homogeneous and pleasant manner. Conversely, the prismatic honeycomb structure has radiation transmission properties which are similar to those of a glazed screen and completely inadequate to obtain a good antibacterial and antiviral effect.

### EXPERIMENTAL SECTION

The antibacterial and antiviral activity of the lamp 1 of the invention on surfaces and on the air of the environment was tested as shown below.

### ANTIBACTERIAL ACTIVITY

### For evaluating antimicrobial activity on surfaces:

Petri dishes were inoculated with the bacterial culture (1.5×10⁴ CFU/ml). The following bacterial cultures were used: *Pseudomonas aeruginosa* ATCC 27853; *Escherichia coli* ATCC 8739; *Staphylococcus aureus* ATCC 43300; *Salmonella typhimurium* ATCC 23853; *Klebsiella pneumoniae* ATCC BAA-1705.

The inoculant was distributed on the plate with a sterile spatula in a laminar flow hood until dry. The inoculated plates were then placed 2 meters below the sanitizing lamp 1 of the invention and exposed to electromagnetic radiation (400-410 nm) for 12 hours.

The positive controls were placed in the same environment but were covered with aluminum foil to avoid exposure to radiation.

At the end of the exposure, both the treated plates as well as the positive controls were incubated for 48 hours at 36°C, then the colonies formed were counted.

The results are shown in Table II below.

**Table II - Logarithmic reduction of microbial load (95% confidence interval)**

| *S. typhimurium* | *K. pneumoniae* | S. *aureus* | *E. coli* | P. *aeruginosa* |
|---|---|---|---|---|
| 2.93 (2.44-3.40) | 2.30 (2.14-2.46) | 3.98 (3.78-4.12) | 3.83 (3.17-4.50) | 3.86 (3.22-4.48) |

### For evaluating the antimicrobial activity on the air of the environment:

Before and immediately after the irradiation with the lamp 1 of the invention, 2 m³ were sucked from the treatment environment, isolated from the outside, by means of a Sas Microflow ALPHA Aquaria apparatus, at a rate of 120 L/min, therefore the air was sent to 60 mm diameter Petri dishes, which were then incubated for 48 hours at 36°C, then the colonies formed were counted.

This operation was repeated for 9 days distributed over two weeks.

The air test was based on normal environmental contamination (non-specific contamination), i.e., without specific inoculation.

The results are shown in Table III.

**Table III**

| **sample** | **Pre-treatment (CFU)** | **Post-treatment (CFU)** |
|---|---|---|
| 1 | 98 | 15 |
| 2 | 71 | 16 |
| 3 | 21 | 12 |
| 4 | 33 | 13 |
| 5 | 40 | 17 |
| 6 | 30 | 1 |
| 7 | 80 | 11 |
| 8 | 107 | 16 |
| 9 | 52 | 20 |

The average percentage reduction in non-specific microbial contamination, calculated on the 9 samples taken, was 72.8%.

### ANTIVIRAL ACTIVITY

A multiwell plate was inoculated with SARS-COV2 viral particles (Lot VMR -SARS COV2 VERO E6 C1008 _28042020, ATCC CRL-1586).

The plate was inoculated with 100 µL of viral suspension, having a concentration of 107.2 TCID50/mL. The TCID50% assay is used to quantify the viral titer by determining the concentration at which 50% of the infected cells exhibit a cytopathic effect (CPE).

The 30x30 cm module provided with 17 blue LEDs 7 was placed at a distance of 40 cm from the inoculated multiwell plate.

The wells were irradiated with the lamp 1 according to the invention for a time of 2 hours; the measured irradiance value was about 3000 microWatt/cm².

The collected suspensions were used to inoculate a 48-well plate into which the VERO E6 cell cultures were fixed. Subsequent decimal dilutions were inoculated for a total of 10 dilutions. Each dilution was inoculated into 4 wells.

The plates were incubated for 3 days at 37°C ± 2°C under a humidified atmosphere with 5% CO₂.

The residual viral activity was assessed by determining the Tissue Culture Effective Dose 50% (TCID50%). Viral titers were determined according to the method developed by Spearman-Karber.

The data are shown in table IV below.

**Table IV - Virucidal activity of the sanitizing lamp according to the invention**

| **Test** | **Susp. Log Viral TCID50% Not treated** | **Susp. Log Viral TCID50% Treated** | **Log reduction TCID50%** |
|---|---|---|---|
| 1 | 4.25 | 1.50 | 2.75 |
| 2 | 4.25 | 1.50 | 2.75 |
| 3 | 4.50 | 1.50 | 3.0 |

A percentage reduction in viral load of 99.85% was found after a treatment with a radiation dose at a wavelength of 400-410 nm of 21.6 J/cm² (dose obtained by multiplying the irradiance in W by the time in seconds).

From what stated above, the advantages of the sanitizing lamp according to the invention appear evident.

The lamp 1 allows sanitizing a closed environment both with respect to the bacterial load and with respect to the viral load (in particular, SARS-COV2 virus).

The fact of being capable of sanitizing even in the presence of one or more persons allows a continuous sanitization of the environment, thus obtaining a high and prolonged level of microbial abatement over time.

The coupling according to a predetermined arrangement of the white LEDs, preferably at a temperature of 3000 degrees Kelvin, to the blue LEDs and the arrangement of the diffuser screen allow creating a diffused, pleasant lighting, without thereby generating eye problems to the persons subjected to such a radiation.

At the same time, the type of diffuser screen used in the present invention provides an adequate shielding combined with a high light transmission at a wavelength of 400-410 nm.

It is apparent that only some particular embodiments of the present invention have been described, to which those skilled in the art will be able to make all changes required to adapt it to particular applications, without departing from the scope of protection of the present invention.

## Claims

1. A sanitizing lamp (1, 101) comprising:
- at least one lighting module (2) equipped with a plurality of white light LED sources (6) and a plurality of blue light LED sources (7);
- a diffuser screen (3),
- a frame (4) and
- a suspension element (5),
**characterized in that** the diffuser screen (3) consists of a sheet of transparent plastic material having a refractive index between 1.4 and 1.6, a light transmittance greater than 90%, and a haze <0.600%, and comprises a smooth outer face (3a) and an inner face (3b) with a hexagonal honeycomb structure.

2. The sanitizing lamp (1, 101) according to claim 1, wherein said diffuser screen (3) consists of a polymethylmethacrylate sheet with a thickness between 2 and 4 mm.

3. The sanitizing lamp (1, 101) according to claim 1 or 2, wherein said hexagonal honeycomb structure consists of hexagons (14) placed side-by-side, wherein each hexagon (14) has a side (A) of a length between 0.8 and 0.9 mm and a profile flared towards the inside with a depth (E) between 0.4 and 0.5 mm.

4. The sanitizing lamp (1, 101) according to any one of claims 1 to 3, wherein the frame (4) comprises an edge (4a) and a support plate (4b), wherein the diffuser screen (3) is fastened at said edge (4a) and wherein between said diffuser screen (3) and said lighting module (2) there is a spacing (13), with a height (F) between 3 and 4.5 cm, or of about 4 cm.

5. The sanitizing lamp (1, 101) according to any one of claims 1 to 4, wherein the lighting module (2) comprises a power supply circuit (8) for the white light LED sources (6) and a power supply circuit (9) for the blue light LED sources (7), said circuits (8, 9) and said LED sources (6, 7) being supported on a panel (10) made of insulating material, fastened in a removable manner to a heat-dissipating plate (12).

6. The sanitizing lamp (1, 101) according to claim 5, wherein the heat-dissipating plate (12) consists of an aluminum sheet with a thickness of about 3 mm.

7. The sanitizing lamp (1, 101) according to any one of claims 1 to 6, wherein the blue light LED sources (7) emit light at a wavelength between 400 and 410 nm, with a peak at 405 nm, and are preferably **characterized by** a minimum radiometric flux between 900 and 1000 mW, which may be driven in current so as to obtain an effective radiometric flux between 1000 and 1500 mW, and are preferably configured to generate a lighting cone with a vertex angle of about 130°.

8. The sanitizing lamp (1, 101) according to any one of claims 1 to 7, wherein the light of the white light LED sources (6) has a temperature of 3000 degrees Kelvin.

9. The sanitizing lamp (1, 101) according to any one of claims 1 to 8, wherein the lighting module (2) comprises a plurality of lighting units (2a) consisting of four white light LED sources (6), arranged at the vertices of a square, and of a blue light LED source (7) arranged in the center of the square, wherein, preferably, the side (C) of the square has a length of about 35 mm.

10. The sanitizing lamp (1, 101) according to any one of claims 4 to 9, wherein the distance (D) of the blue light LED sources (7) arranged further externally from the edge (4a) is of 5 cm or more, or about 6 cm.

11. The sanitizing lamp (1, 101) according to any one of claims 1 to 10, wherein the at least one lighting module (2) comprises 64 white light LED sources (6) and 17 blue light LED sources (7).

12. The sanitizing lamp (101) according to any one of claims 1 to 11, wherein the suspension element (5) comprises a stem (5a) and a fastening element (5b) for fastening it to a ceiling of a room and wherein, preferably, the stem (5a) has a length Lg in meters given by the equation Lg = L - x, with x being between 2.40 and 2.90 meters.

13. The sanitizing lamp (1) according to any one of claims 1 to 11, said lamp being selected from a ceiling lamp, a table lamp, a floor lamp, and a wall lamp.
